# EUROPÄISCHE PATENTANMELDUNG

(11) **EP 0 972 489 A2**
(43) Veröffentlichungstag der Anmeldung: **19.01.2000**
(21) Anmeldenummer: 99112982.6
(22) Anmeldetag: 05.07.1999
(51) Int. Cl.: A61B 5/00

(54) **Verfahren zur Ermittlung des im Rahmen des Parkinson-Syndroms bzw. der Parkinson-Krankheit relevanten klinischen Zustands eines Patienten, sowie System hierfür**

(30) Priorität: 16.07.1998 DE 19832053
(71) Anmelder: SIEMENS AKTIENGESELLSCHAFT, 80333 München (DE)
(72) Erfinder: Abraham-Fuchs, Klaus, Dipl.-Phys., 91058 Erlangen (DE); Birkhölzer, Thomas, Dr., 91085 Weisendorf (DE); Schmidt, Kai-Uwe, Dr., 91052 Erlangen (DE); Schmidt, Volker, Dr., 91054 Erlangen (DE)

(57) **Zusammenfassung**

Verfahren zur Ermittlung des im Rahmen des Parkinson-Syndroms bzw. der Parkinson-Krankheit relevanten klinischen Zustands eines Patienten, wobei ein oder mehrere meßtechnisch erfaßbare, krankheitsbedingt beeinflußte Parameter ermittelt werden, auf denen basierend unter Verwendung eines rechnergestützten Analyse- und Bewertungssystems der klinische Zustand bestimmt wird.

## Beschreibung

Die Erfindung betrifft ein Verfahren zur Ermittlung des im Rahmen des Parkinson-Syndroms bzw. der Parkinson-Krankheit relevanten klinischen Zustands eines Patienten.

Vor allem im höheren Alter treten Erkrankungen auf, deren Ursache Mängel oder Konzentrationsschwankungen bestimmter körpereigener Stoffe sind. Eine solche Krankheit ist die Parkinson-Krankheit, bei der es sich um eine neurologische Erkrankung handelt. Die Patienten bemerken hierbei eine Beeinträchtigung der willkürlichen und unwillkürlichen Beweglichkeit, eine Verlangsamung der Bewegungsabläufe, eine Steifigkeit der Muskulatur, vorwiegend in den Gliedmaßen oder ein Zittern der Arme und Beine, vorwiegend in Ruhe. Die drei Hauptsymptome dieser Erkrankung sind Akinese (Bewegungshemmung), Rigor (Erstarrung), Tremor (Zittern). Die genaue Ursache der Erkrankung ist bis heute nicht geklärt, es handelt sich hier um eine langsam fortschreitende, degenerative Erkrankung bestimmter Nervenzellen, in den Basalganglien (Ansammlung von Nervenzellen im Groß-, Zwischen- und Mittelhirn). Die miteinander verbundenen Nervenzellen können durch chemische Botenstoffe, sogenannte Transmitter, Informationen austauschen oder weiterleiten. Dabei benutzen die einzelnen Kerngebiete in ihren Nervenbahnen oft unterschiedliche Botenstoffe. So gibt es Transmitter, die andere Nervenzellen stimulieren, und solche, die hemmende Einflüsse ausüben. Dem Botenstoff Dopamin, der zur Impulsübertragung von einer Nervenzelle zur nächsten in andere Systeme des Gehirns zuständig ist, kommt dabei besondere Bedeutung zu. Neben einem Mangel an Dopamin findet auch eine Verschiebung im Verhältnis zu anderen Transmitterstoffen statt. Am meisten gestört ist beim Parkinson-Syndrom das Gleichgewicht zwischen Dopamin und Acetylcholin.

Durch Verminderung von Dopamin kommt es zu einer funktionellen Übersteuerung von Acetylcholin, auf das das Zittern und die erhöhte Muskelspannung zurückgehen.

Aufgrund der geschilderten Krankheitsursachen ist eine direkte Behandlung nur durch Medikamente möglich. Bei den verwendeten Medikamenten handelt es sich um Präparate, die den Dopaminmangel ausgleichen sollen, die aber bei Überdosierung starke Nebenwirkungen besitzen. Eine optimale Dosierung dieser Medikamente ist schwierig. Zum einen ist die Wirkung stark personenabhängig. Ferner hat die Medikamentenwirkung einen zeitlichen Verlauf (Anreicherungsphase, Abbauphase), der berücksichtigt werden muß. Der Bedarf und die Wirkung von Wirkstoffen sind kontextabhängig, zum Beispiel von der Aktivität des Patienten oder dergleichen. Schließlich beeinflussen sich die verschiedenen Wirkmechanismen unterschiedlicher Medikamente gegenseitig, so daß einerseits die Wirkung nicht hinreichend ist, andererseits auch Nebenwirkungen auftreten können. Letztlich können die für den Krankheitszustand relevanten Größen, z. B. der Dopaminspiegel im Gehirn im Falle der Parkinson-Erkrankung nicht direkt gemessen werden.

Der behandelnde Arzt ist infolgedessen auf eine experimentelle Medikamentation angewiesen. Er ist gezwungen, nach dem 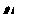 try and error"-Prinzip zu handeln. Dem Patienten wird eine vorbestimmte erste Dosis verschrieben, wonach die Symptome abgewartet werden, d.h., es wird die Wirkung dieser Erstmedikamentation beobachtet. Abhängig hiervon erfolgt dann iterativ eine Anpassung der Dosis.

Aus der DE 196 37 383 A1 ist ein Verfahren zur Überwachung einer Person bekannt, bei der auf der Basis von körpernah zu tragenden Sensoren Daten ermittelt werden, die mit einem üblichen Tagesablauf der zu überwachenden Person entsprechenden Daten verglichen werden. Beim Ausbleiben typischer Daten erfolgt eine Anzeige, um die zu überwachende Person beispielsweise an eine auszuführende Tätigkeit zu erinnern.

Aus der DE 196 14 255 C1 ist ein Verfahren bekannt, bei dem, sei es zu Zwecken der Therapie oder der pharmazeutischen Forschung, Befindlichkeitswerte eines Patienten nach der Einnahme eines Medikaments registriert werden, so daß die Wirkung des Medikaments aufgezeichnet und reproduziert werden kann.

Die DE 43 31 018 A1 betrifft ein Verfahren zur automatischen Bewertung eines Krankheitstyps anhand von Proteinfraktionsdaten, d.h. es handelt sich um ein biochemisches Analyseverfahren.

Der Erfindung liegt das Problem zugrunde, eine Verfahren anzugeben, mittels welchem der klinische Zustand des Patienten hinreichend aussagekräftig bestimmbar ist, so daß die Medikamentation auf einer dem tatsächlichen klinischen Zustand angenäherten Grundlage erfolgen kann.

Zur Lösung diese Problems ist bei einem Verfahren der eingangs genannten Art erfindungsgemäß vorgesehen, daß ein oder mehrere meßtechnisch erfaßbare, krankheitsbedingt beeinflußte Paramter erfaßt werden, auf denen basierend unter Verwendung eines rechnergestützten Analyse- und Bewertungssystems der klinische Zustand bestimmt wird.

Das erfindungsgemäße Verfahren basiert darauf, daß mindestens ein (vorzugsweise mehrere verschiedene) Parameter gemessen wird, welcher in seiner Ausprägung vom Grad der Erkrankung bzw. vom momentanen klinischen Zustand des Patienten abhängig ist. Im Falle einer Parkinson-Erkrankung kann erfindungsgemäß als Paramenter unter Verwendung eines oder mehrerer Sensorelemente die Bewegung eines oder mehrerer Körperteile oder Sinnesorgane, z.B. die Pupillenfolgefähigkeit des Patienten erfaßt werden. Wie bereits beschrieben,äußert sich die Parkinson-Erkrankung schwerpunktmäßig in einer Beeinflussung der Bewegungsmöglichkeiten und Motorik des Patienten. Je fortgeschrittener die Krankheit ist, desto stärker sind die auftretenden Störungen. Der Patient kann in solchen Fällen stärker zittern als bei einem normalen Dopaminspiegel, die Muskulatur kann steifer sein. Die Erfassung dieser meßtechnischen Werte läßt unter Verwendung des Analyse- und Bewertungssystems, das eine Rechnereinrichtung mit entsprechender Software umfaßt, eine Aussage hinsichtlich des klinischen Zustands des Patienten zu, das heißt, der Zustand kann aussagekräftig bewertet werden. Es ist also eine Ermittlung der aktuellen Schwere der Krankheit möglich. Diese Aussage bzw. Bewertung kann dann Grundlage für die anschließende Medikamentation sein. Das System berücksichtigt den bisher bekannten Krankheitsprozeß bzw. den allgemeinen Verlauf der Parkinson-Erkrankung, ist also spezifisch für diese Krankheit.

Alternativ oder zusätzlich zu der Erfassung etwaiger Bewegungen von Körperextremitäten kann auch die Bewegung oder Tätigkeit eines Sinnesorganes wie dem Auge erfaßt und verarbeitet werden. Hierzu kann beispielsweise mittels eines Eyescanners die Lidschlagfrequenz oder die Pupillenfolgefähigkeit ermittelt werden, wobei diese Bewegungen ebenfalls bei Vorliegen der Parkinson-Krankheit stark verlangsamt erfolgen.

Anstelle dieses Parameters, selbstverständlich auch zusätzlich zu diesem, kann ein quasi indirekter Parameter erfaßt werden, welcher ebenfalls in seiner Ausprägung krankheitsbedingt beeinflußt ist. Bei einem solchen Parameter kann-es sich um eine Meßgröße zur Bestimmung der motorischen Aktivität oder der motorischen Steuerungsfähigkeit des Patienten handeln. Hier können erfindungsgemäß aus einer bewußten Bewegungs- und/oder Sprachtätigkeit ermittelbare Meßwerte verwendet werden. Der Patient wird beispielsweise aufgefordert, auf einer entsprechenden Schreibeinrichtung ein Wort oder dergleichen zu schreiben. Diese elektronische Schreibeinrichtung ist derart ausgestaltet, daß die Bewegung des Schreibwerkzeugs auf der Unterlage elektronisch ermittelt und in Meßwerte umgesetzt werden kann, welche dann verarbeitet werden. Die Bewegungs- oder Sprachtätigkeit des Patienten hängt ebenfalls vom jeweiligen Momentanzustand ab. Ist ein hoher Dopaminmangel gegeben, nimmt die Bewegungstätigkeit beispielsweise schneller ab als bei ausgeglichenem Dopaminspiegel. Gleiches gilt betreffend die Sprachfähigkeit des Patienten. Auch diese Meßwerte können in die Analyse und Bewertung mit eingehen. Das System verarbeitet diese Meßwerte, gegebenenfalls unter Verwendung weiterer bereits bekannter Meßwerte wie der Historie der Medikamenteneinnahme, der Historie der Aktivität des Patienten, der Historie der Nahrungsaufnahme oder der Historie des Krankheitsprozesses, um basierend hierauf den relevanten klinischen Zustand zu bestimmen.

Dabei hat es sich als zweckmäßig erwiesen, wenn erfindungsgemäß zusätzlich zu den meßtechnisch ermittelten Parametern wenigstens ein subjektiver, insbesondere das subjektive Empfinden des Patienten beschreibender Parameter ermittelt und der Recheneinrichtung gegeben wird, welche im Rahmen der Analyse verarbeitet wird. Dieser subjektive Parameter ist ebenfalls äußerst aussagekräftig. Hier kann beispielsweise das subjektive Befinden, die subjektive Bewegungsfähigkeit, die letzten Aktivitäten des Patienten, etwaige Schwindelgefühle etc. oder aber auch Konzentrationsübungen und deren Verlauf mitberücksichtigt werden.

Zur Ermittlung des klinischen Patientenzustands bzw. als Maß für diesen, kann erfindungsgemäß der mittels des Systems basierend auf dem oder den Parametern abschätzbare Dopaminspiegel verwendet werden. Das System ist gemäß dieser Erfindungsausgestaltung in der Lage, den Dopaminspiegel abzuschätzen und basierend auf diesem den aktuellen klinischen Zustand des Patienten zu bestimmen. Werden also Meßwerte erhalten, die auf einen niedrigen Dopaminspiegel zurückzuführen sind, kann dieser als entsprechendes Maß für den klinischen Patientenzustand abgeschätzt werden. Alternativ oder zusätzlich hierzu kann erfindungsgemäß als Maß für den klinischen Patientenzustand ein basierend auf den ermittelten Parametern bestimmbares Maß für die objektive Bewegungsunfähigkeit verwendet werden. Hierbei werden also die ermittelten Parameter, beispielsweise die Parameter, die aufgrund von Körperteilbewegungen erfaßt werden, in ihrer Qualität bemessen und dieses bestimmte Maß als Grundlage für die Beurteilung des klinischen Patientenzustands herangezogen. Daneben ist es erfindungsgemäß auch möglich, den klinischen Patientenzustand in Form eines Maßes für das subjektive Empfinden des Patienten, welches basierend auf den eingegebenen Parametern bestimmbar ist, zu verwenden. Alle drei Grundlagenmaße können zweckmäßigerweise als Zahlenwerte ausgegeben werden, die dem behandelnden Arzt eine einfache Kenntnis des ermittelten aktuellen klinischen Zustands ermöglichen.

Ferner kann erfindungsgemäß basierend auf den ermittelten Parametern und/oder dem bestimmten klinischen Zustand mittels eines Simulationsmodells der zukünftige zeitliche Verlauf des klinischen Patientenzustands simuliert werden, wobei zweckmäßigerweise hierbei auch die Wirkung eines oder mehrerer, im Rahmen einer Therapie verabreichbarer Medikamente auf den klinischen Patientenzustand simulierbar ist. Die Simulationsmöglichkeit bringt den beachtlichen Vorteil, daß vor der tatsächlichen Medikamentengabe überprüft werden kann, ob und wie dasjenige Medikament, welches gegeben werden soll, therapierend wirkt oder keinerlei Wirkung zeigt. Im Hinblick darauf, daß das Modell eine Reihe medikamentenspezifischer Daten beinhaltet, die im Rahmen der Simulation berücksichtigt werden können, kann so ein eine hinreichende Aussage ermöglichendes Simulationsergebnis erhalten werden, das dem Arzt hilfreich ist. Vorteilhaft ist ferner die erfindungsgemäße Weiterbildung, gemäß welcher mittels des Simulationsmodelles in einem Optimierungsverfahren eine optimale Therapie insbesondere Medikamentendosierung, also eine Therapieempfehlung, bestimmt wird, was zweckmäßigerweise automatisch mittels des Systems, und unter Zugrundelegung erhaltener Simulationsergebnisse erfolgt.

Gemäß einer zweckmäßigen Weiterbildung des Erfindungsgedankens kann vorgesehen sein, daß das Analyse- und Bewertungssystem und/oder das Simulationsmodell im Rahmen des patientenspezifischen Betriebs adaptiv an den individuellen Patienten angepaßt werden. Diese Erfindungsausgestaltung ermöglicht eine kontinuierliche Anpassung des Systems bzw. Modells, welches von einem a-priori-Zustand zu Beginn seines Einsatzes ausgeht, so daß sich das Modell quasi selbständig an den Patienten und dessen klinischen Zustand anpaßt und die mit ihm treffbare Analyse und Bewertung mit Fortschreiten der Zeit immer genauer wird. Als ein derartiges System und/oder Modell können einsetzbar sein:
- neuronales Netz mit den parameterspezifischen Meßwerten und der Medikamentendosierung als Eingang und dem Stoffspiegel, beispielsweise dem Dopaminspiegel als Ausgang,
- Nichtlineare Beobachter mit internem Zustand 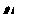 Stoffspiegel" (z. B. Dopaminspiegel) und den parameterspezifischen Meßwerten als Meßgröße,
- Causal Probalistic Network mit Stoffspiegel (Dopaminspiegel), parameterspezifischen Meßwerten, Medikamentendosierung und Aktivität als Knoten,
- ein Expertensystem.

Neben dem Verfahren betrifft die Erfindung ferner ein System zur Ermittlung des im Rahmen des Parkinson-Syndroms bzw. der Parkinson-Erkrankung relevanten klinischen Zustands eines Patienten. Dieses System zeichnet sich erfindungsgemäß dadurch aus, daß ein rechnergestütztes Analyse- und Bewertungssystem vorgesehen ist, welchem ein oder mehrere meßtechnisch erfaßbarer, krankheitsbedingter Parameter zuführbar ist, der mittels des Systems verarbeitbar und auf dem basierend der klinische Zustand mittels des Systems bestimmbar ist.

Das System kann erfindungsgemäß ferner ein oder mehrere Sensorelemente zum Messen von Bewegungen eines oder mehrerer Körperteile oder Sinnesorgane umfassen, wobei die ermittelbaren Meßwerte die Parameter darstellen. Zusätzlich oder alternativ können ferner ein oder mehrere Mittel zum erfassen einer bewußten Bewegungs- und/oder Sprachtätigkeit des Patienten vorgesehen sein, wobei die ermittelbaren Meßwerte die gegebenenfalls weiteren Parameter darstellen.

Der Rechnereinrichtung können erfindungsgemäß zusätzlich zu den meßtechnisch ermittelbaren Parametern wenigstens ein subjektiver, insbesondere das subjektive Empfinden des Patienten beschreibender Parameter zuführbar sein, welcher im Rahmen der Analyse verarbeitbar ist. Wie beschrieben kann es sich bei diesen Parametern um das Anamneseergebnis handeln.

Um neben der Kenntnis des aktuellen klinischen Zustands des Patienten eine grobe Angabe hinsichtlich der weiteren Therapie bzw. Medikamentation zu erhalten, kann erfindungsgemäß ein Simulationsmodell vorgesehen sein, das im Stande ist, neben dem zukünftigen zeitlichen Verlauf des klinischen Patientenzustands auch die Wirkung eines verabreichbaren Medikaments auf den Patientenzustand zu simulieren und das jeweilige Simulationsergebnis auszugeben. Auch kann eine optimale Therapie, insbesondere Medikamentendosierung mittels des Simulationsmodelles in einem Optimierungsverfahren bestimmbar sein. Das System und/oder das Modell können im Rahmen des Betriebs adaptiv an den individuellen Patienten anpaßbar sein. Schließlich kann das System bzw. das Modell ein neuronales Netz, ein nichtlinearer Beobachter, ein Causal Probalistic-Network oder ein Expertensystem sein. Auf einem in Form eines Monitors oder Displays ausgebildeten Anzeigemittel können schließlich die ermittelten Ergebnisse und Daten angezeigt werden.

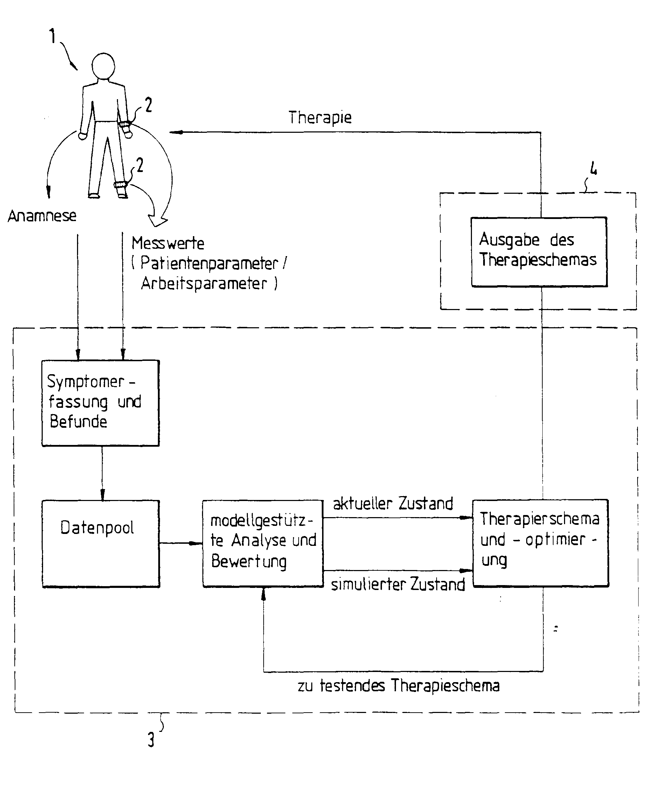

Weitere Vorteile, Merkmale und Einzelheiten der Erfindung ergeben sich aus dem im folgenden beschriebenen Ausführungsbeispiel sowie anhand der Zeichnung.

In dieser ist das erfindungsgemäße System in Form einer Prinzipskizze dargestellt. Gezeigt ist ein Patient 1, der am Parkinson-Syndrom leidet. Diese Krankheit äußert sich in Form von Akinese (Bewegungshemmung), Rigor (Erstarrung) und Tremor (Zittern) . Sämtliche Symptome wirken also in Form einer motorischen Störung. Mittels Sensorelementen 2 wird die Bewegung des Patienten 1, welche letztlich ein Maß für den Krankheitszustand ist gemessen. Die Meßwerte werden für diese Parameter aufgenommen. Ferner werden weitere Meßwerte im Rahmen der Symptomerfassung erstellt. Bei diesen handelt es sich um 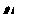 indirekte" Meßwerte, die zu einer bestimmten Bewegungs- und/oder Sprachtätigkeit des Patienten ermittelt werden. Der Patient wird hierbei aufgefordert, ein bestimmtes Wort zu sprechen oder ein Wort zu schreiben, wobei meßtechnisch das Sprechen bzw. Schreiben ausgewertet wird. Relevant ist hierbei beispielsweise die Sprachgeschwindigkeit, die Lautstärke, Betonung etc., beim Schreiben beispielsweise die Zeit bis zur Beendigung des Wortes, der Schreibdruck, die Schreibgeschwindigkeit etc. Da die Parkinson-Krankheit die Motorik und Bewegungsmöglichkeit beeinflußt, sind auch diese Meßwerte relevant.

Zusätzlich zu diesen meßtechnisch ermittelbar Werten tragen zur Symptomerfassung bzw. als aussage- und verarbeitungswerte Befunde auch Anamnesedaten bei. Im Rahmen dieser Anamnese wird der Patient nach seinem subjektiven Befinden, nach seiner subjektiv eingeschätzten Bewegungsfähigkeit oder den letzten von ihm durchgeführten Aktivitäten befragt. Diese subjektiven Daten werden zusammen mit den Meßwerten als Symptome und Befunde dem Datenpool zugeführt, aus welchem im Rahmen der rechnergestützten Analyse und Bewertung geschöpft werden kann. Diese Analyse und Bewertung erfolgt mittels eines softwaretechnischen Analyse- und Bewertungssystems. Dieses System dient dazu, aus den zugänglichen Größen, die im Datenpool zusammengefaßt sind, den aktuellen klinischen Zustand des Parkinson-Patienten zu bestimmen bzw. zu bewerten. Im Datenpool sind neben den aufgenommenen Ist-Symptomen bzw. Befunden auch die Historie der Medikamenteneinnahme, die Historie der Patientenaktivität sowie der Nahrungsaufnahme und die Historie des Krankheitsprozesses vereint. Je größer der Datenpool ist, desto exakter kann analysiert und bewertet werden. Das System selbst ist bevorzugt ein lernendes Modell, d. h., es verwertet die jeweils aufgenommenen neuen Daten zur eigenen Optimierung.

Das fertige Analyse- und Bewertungsergebnis gibt den aktuellen Zustand wieder. Hierauf basieren kann ein Therapieschema rechnerseitig erstellt werden. Dieses Therapieschema kann im Rahmen einer Therapieoptimierung einem Simulationsmodell unterworfen werden, welches zur Simulation des klinischen Zustands des Patienten nach Durchführung der Therapie ausgebildet ist. Anhand dieser Simulationsmöglichkeit kann das Therapieschema weiter optimiert werden, wenn sich im ersten erstellten Therapieschema basierend auf dem aktuellen Zustand innerhalb der Simulation Schwierigkeiten ergeben. Dabei ist dem Modell neben den bereits genannten Daten auch das Wirkungsprofil der unterschiedlichen verabreichbaren Medikamente wie auch die gegenseitige Wirkungsbeeinflussung der einzelnen Medikamente bekannt. Dies ist erforderlich, um einerseits in Abhängigkeit des klinischen Zustands der Patienten und dessen körperlicher Situation das oder diejenigen Medikamente auszuwählen, die für ihn überhaupt in Frage kommen, zum anderen ist es zwingend erforderlich, Kenntnis über etwaige Nebenreaktionen zu bekommen, um diese auszuschließen. Daneben sollte auch bekannt sein, ob etwaige andere Medikamente verabreicht werden, die außerhalb des Therapieschemas für die Parkinson-Erkrankung stehen, jedoch möglicherweise in nachteiliger Form mit dem zu verabreichenden Medikamenten im Rahmen der Parkinson-Therapie wechselwirken. Sämtliche Vorgänge erfolgen in einer Rechnereinrichtung 3, der die erforderlichen Meßwerte und Anamnesedaten zuführbar sind, im ersten Fall automatisch durch eine entsprechende Kommunikationsverbindung zu den Sensorelementen 2, im letzten Fall z. B. manuell durch Eingabe entsprechender Informationen über eine Tastatur oder dergleichen.

Nach Ermittlung eines durchzuführenden jeweiligen Ergebnisses, z.B. das Therapieschemas wird dieses letztlich an einem Anzeigemittel 4, z. B. einen Monitor ausgegeben, wonach die eigentliche Therapie und Medikamentverabreichung erfolgt. Der tatsächliche Therapieerfolg wird dann wiederum anhand der Meßwerte/Anamnese ermittelt.

Das System ermöglicht in hinreichend aussagekräftiger Form die Bestimmung des aktuellen klinischen Patientenzustands und bietet ferner eine zweckmäßige Simulationsmöglichkeit. Erforderlich hierfür ist insoweit lediglich eine mit dem softwaremäßigen Analyse- und Bewertungssystem und dem Simulationsmodell belegte Rechnereinrichtung, welcher die entsprechenden Sensorelemente zugeordnet sind. Die Anamnesedaten kann der Patient, sofern es sein Zustand zuläßt, selbst eingeben. Lediglich erforderlich ist noch eine Ausgabeeinrichtung, beispielsweise in Form eines Monitors. Ersichtlich kann dieses System beim Patienten selbst zum Einsatz kommen, der dieses dann selbst bedienen kann. Erforderlich ist lediglich, daß er die entsprechenden Sensorelemente anbringt und die geforderten Anamnesedaten eingibt, alles andere erfolgt automatisch rechnerseitig. Die zu verabreichenden Medikamente und deren Dosierung kann der Patient dem ausgegebenen Therapieschema am Monitor oder dergleichen selbst entnehmen und sich selber zuführen. Eine ärztliche Beaufsichtigung ist im wesentlichen nicht mehr erforderlich.

## Patentansprüche

1. Verfahren zur Ermittlung des im Rahmen des Parkinson-Syndroms bzw. der Parkinson-Krankheit relevanten klinischen Zustands eines Patienten, bei dem wenigstens ein meßtechnisch erfaßbarer, krankheitsbedingt beeinflußter Parameter erfaßt wird, auf dessen Basis unter Verwendung eines rechnergestützten Analyse- und Bewertungssystems der klinische Zustand des Patienten bestimmt wird.

2. Verfahren nach Anspruch 1, bei dem als Parameter Bewegungen eines oder mehrerer Körperteiles oder Sinnesorgane des Patienten unter Verwendung wenigstens eines oder mehrerer Sensorelemente erfaßt werden.

3. Verfahren nach Anspruch 1 oder 2, bei dem als Parameter aus einer bewußten Bewegungs- und/oder Sprachtätigkeit des Patienten ermittelbare Meßwerte erfaßt werden.

4. Verfahren nach einem der Ansprüche 1 bis 3, bei dem zusätzlich zu den meßtechnisch ermittelten Parametern wenigstens ein subjektiver, insbesondere das subjektive Empfinden des Patienten beschreibender Parameter erfaßt und verwendet wird.

5. Verfahren nach einem der vorangehenden Ansprüche, bei dem der klinische Zustand des Patienten auf Basis des basierend auf wenigstens einem Parameter geschätzten Dopaminspiegels des Patienten bestimmt wird.

6. Verfahren nach einem der vorangehenden Ansprüche, bei dem der klinische Zustand des Patienten auf Basis eines basierend auf wenigstens einem Parameter ermittelten Maßes für die objektive Bewegungsfähigkeit des Patienten ermittelt wird.

7. Verfahren nach einem der vorangehenden Ansprüche, bei dem der klinische Zustand des Patienten auf Basis eines basierend auf wenigstens einem Parameter ermittelten Maßes für das subjektive Empfinden des Patienten ermittelt wird.

8. Verfahren nach einem der vorangehenden Ansprüche, bei dem zur Angabe des jeweiligen Maßes für den klinischen Zustand des Patienten Zahlenwerte ausgegeben werden.

9. Verfahren nach einem der vorangehenden Ansprüche, bei dem basierend auf den erfaßten Parametern und/oder dem ermittelten klinischen Zustand mittels eines Simulationsmodells der zukünftige zeitliche Verlauf des Zustandes des Patienten simuliert wird.

10. Verfahren nach einem der vorangehenden Ansprüche, bei dem basierend auf den erfaßten Parametern und/oder dem ermittelten klinischen Zustand des Patienten mittels eines Simulationsmodells die Wirkung eines oder mehrerer, im Rahmen einer Therapie dem Patienten verabreichbarer Medikamente auf den Zustand des Patienten simulierbar ist.

11. Verfahren nach Anspruch 10, bei dem mittels des Simulationsmodelles in einem Optimierungsverfahren eine optimale Therapie, insbesondere Medikamentendosierung, bestimmt wird.

12. Verfahren nach einem der vorangehenden Ansprüche, bei dem das Analyse- und Bewertungssystem und/oder das Simulationsmodell im Rahmen des Betriebs des Systems adaptiv an den jeweiligen individuellen Patienten angepaßt werden/wird.

13. Verfahren nach einem der vorangehenden Ansprüche, bei dem als System und/oder Simulationsmodell ein neuronales Netz, ein Nichtlinearer Beobachter, ein Causal Probalistic Network oder ein Expertensystem verwendet wird.

14. System zur Ermittlung des im Rahmen der Parkinson-Syndroms bzw. der Parkinson-Krankheit relevanten klinischen Zustands eines Patienten, welches ein rechnergestütztes Analyse- und Bewertungssystem (3) aufweist, welchem wenigstens ein meßtechnisch erfaßbarer, krankheitsbedingt beeinflußter Parameter zuführbar ist, den das System verarbeitet und auf dessen Basis es den klinischen Zustand des Patienten bestimmt.

15. System nach Anspruch 14, das wenigstens ein Sensorelement (2) zum Messen von Bewegungen eines oder mehrerer Körperteile oder Sinnesorgane umfaßt, wobei die mittels des Sensorelementes ermittelten Meßwerte ermittelbaren Meßwerte Parameter darstellen.

16. System nach Anspruch 15 oder 16, das wenigstens ein oder mehrerer Mittel zum Erfassen einer bewußten Bewegungs- und/oder Sprachtätigkeit des Patienten umfaßt, wobei die mittels der Mittel zum Erfassen ermittelten Meßwerte Parameter darstellen.

17. System nach einem der Ansprüche 14 bis 16, welches zusätzlich zu meßtechnisch ermittelten Parametern wenigstens einen subjektiven, insbesondere das subjektive Empfinden des Patienten beschreibenden Parameter erfaßt, welcher im Rahmen der Analyse verarbeitbar ist.

18. System nach einem der Ansprüche 14 bis 17, welches ein Simulationsmodell aufweist, das den zukünftigen zeitlichen Verlauf des klinischen Zustandes des Patienten bestimmt.

19. System nach einem der Ansprüche 14 bis 18, dessen Analyse- und Bewertungssystem ein Simulationsmodell zugeordnet ist, welches dem basierend auf den erfaßten Parametern und/oder dem ermittelten klinischen Zustand des Patienten die Wirkung eines oder mehrerer, im Rahmen einer Therapie verabreichbarer Medikamente auf den klinischen Zustand des Patienten simuliert.

20. System nach Anspruch 19, das anhand des mittels des Simulationsmodells vorhergesagten klinischen Zustandes des Patienten eine Therapieempfehlung, insbesondere eine Medikamentendosierung, bestimmt.

21. System nach einem der Ansprüche 14 bis 20, Analyse- und Bewertungssystem und/oder das Simulationsmodell sich im Rahmen des Betriebs des Systems adaptiv an den jeweiligen individuellen Patienten anpaßt.

22. System nach einem der Ansprüche 14 bis 21, dessen Analyse- und Bewertungssystems und/oder Simulationsmodell ein neuronales Netz, ein Nichtlinearer Beobachter, ein Causal Probalistic Network oder ein Expertensystem ist.

23. System nach einem der Ansprüche 14 bis 22, das Anzeigemittel (4) in Form eines Monitors oder eines Displays aufweist.
